# EUROPÄISCHE PATENTANMELDUNG

(11) **EP 4 614 147 A1**
(43) Veröffentlichungstag der Anmeldung: **10.09.2025**
(21) Anmeldenummer: 24161073.2
(22) Anmeldetag: 04.03.2024
(51) Int. Cl.: G01N 33/543

(54) **IMMUNPRÄZIPITATIONSASSAY MIT NICHT-BLUT-BASIERTEN ANTIKÖRPERN**

(71) Anmelder: Siemens Healthcare Diagnostics Products GmbH, 35041 Marburg (DE)
(72) Erfinder: Schwarz, Herbert, 35102 Lohra (DE); Barten, Roland, 35041 Marburg (DE); Duwe, Christa, 35075 Gladenbach (DE); Muth, Sandra, 35041 Marburg (DE)
(74) Vertreter: Siemens Healthineers Patent Attorneys

(57) **Zusammenfassung**

Die vorliegende Erfindung liegt auf dem Gebiet der in vitro-Diagnostik und betrifft Reagenzien enthaltend nicht-Blutbasierte Antikörper zur Bestimmung eines Analyten in einem Immunpräzipitationsassay und mit einer Sensitivität, die vergleichbar ist mit herkömmlichen Reagenzien, die polyklonale Antikörperseren enthalten.

## Beschreibung

Die vorliegende Erfindung liegt auf dem Gebiet der in vitro-Diagnostik und betrifft Reagenzien enthaltend nicht-Blutbasierte Antikörper zur Bestimmung eines Analyten in einem Immunpräzipitationsassay und mit einer Sensitivität, die vergleichbar ist mit herkömmlichen Reagenzien, die aus polyklonalen Antikörperseren bestehen.

Immunpräzipitationsassays gehören zur Gruppe der homogenen Immunoassays und basieren auf der Wechselwirkung zwischen Antikörpern und nachzuweisendem Antigen. Die entstehenden Immunkomplexe führen zu Aggregaten und hochmolekularen Präzipitaten, die in Lösung einfallendes Licht absorbieren beziehungsweise auf einfallendes Licht streuend wirken oder in Gelen ein verändertes Diffusionsverhalten zeigen. Die durch die Immunaggregate verursachte Trübung eines Reaktionsgemisches verhält sich im Wesentlichen proportional zu der im Reaktionsgemisch enthaltenen Antigenmenge und kann als Anstieg der Extinktion (turbidimetrisch) oder als Zunahme einer Streulichtintensität unter einem festgelegten Winkel gegenüber dem einfallenden Licht (nephelometrisch) quantitativ bestimmt werden. Die optimalen Bedingungen zur Durchführung solcher Immunpräzipitationsassays, wie Puffer, Detergenzien, Neutralsalze, Beschleuniger, Probenverdünnung usw. sind in der Literatur beschrieben und variieren in Abhängigkeit von den jeweils verwendeten Antikörpern und den nachzuweisenden Antigenen. Die Vorteile der immunturbidimetrischen und immunnephelometrischen Verfahren sind relativ einfache Automatisierbarkeit und niedrige Herstellkosten, wodurch sie eine weite Verbreitung in der in vitro-Diagnostik gefunden haben.

Bei der Durchführung von Immunpräzipitationsassays finden aufgrund der optimalen vernetzenden Eigenschaften vor allem tierische Antiseren Anwendung, wie z.B. Seren von Kaninchen, Pferden oder Mäusen, die mit einem Antigen immunisiert wurden, wobei solche Antiseren letztendlich ein polyklonales Gemisch von verschiedenen natürlichen, Blut-basierten Antikörpern, die gegen verschiedene Epitope des Antigens gerichtet sind, enthalten.

Die Nachteile der Verwendung von tierischen Antiseren bestehen einerseits darin, dass Qualitätsschwankungen von in vitro-diagnostischen Produkten unvermeidbar sind, da jedes immunisierte Tier eine individuelle Immunantwort produziert und jeweils nur eine limitierte Menge Antiserum verfügbar ist. Andererseits werden für die Gewinnung der Antiseren viele Tiere benötigt, deren Haltung, Behandlung und Schlachtung strengen tierschutzrechtlichen Vorschriften gerecht werden muss und nicht zuletzt auch ethische Bedenken hervorruft.

Zur Vermeidung dieser Nachteile kommen zunehmend Antikörpermischungen zum Einsatz, die verschiedene rekombinante oder monoklonale Antikörper enthalten, die gegen unterschiedliche Epitope eines Antigens gerichtet sind (sogenannte "multiklonale Antikörpermischungen") und versuchen ein polyklonales Antiserum nachzuahmen. Mittels Hybridom-Technik können monoklonale Antikörper in vitro produziert werden, was den Tierverbrauch im Vergleich zur Herstellung polyklonaler Antiseren deutlich reduziert. Mittels der Phagen-Display Technologie können Antikörperkandidaten in vitro selektiert und anschließend in in vitro-Expressionssystemen produziert werden ("rekombinante Antikörper"), wobei auf den Einsatz von Tieren vollständig verzichtet werden kann. Die Vorteile solcher sogenannter multiklonaler Antikörpermischungen bestehen in der vollständigen Reproduzierbarkeit ihrer Zusammensetzung und größtmöglichem Verzicht auf den Einsatz und Verbrauch von Tieren.

Die Verwendung von Reagenzien, die multiklonale Antikörpermischungen enthalten, ermöglicht die sensitive und spezifische Bestimmung verschiedenster Analyten in verschiedenen Immunoassayformaten, wie z.B. in ELISA-Assays oder in der Durchflusszytometrie. Bei der Verwendung in Immunpräzipitationsverfahren hingegen, insbesondere in turbidimetrischen oder nephelometrischen Immunpräzipitationsverfahren, wird jedoch häufig keine Sensitivität erreicht, die mit der Sensitivität, die durch den Einsatz von tierischen Antiseren erzielt wird, vergleichbar wäre. Dies schließt die tierschonende Anwendung von nicht-Blut-basierten bzw. nicht-tierischen, multiklonalen Antikörpermischungen in Immunpräzipitationsverfahren bislang von einer breiten Anwendung in der in vitro-Diagnostik aus.

Der vorliegenden Erfindung liegt damit die Aufgabe zugrunde, den Vorteil der tierschonenden Anwendung von nicht-Blut-basierten bzw. nicht-tierischen, multiklonalen Antikörpermischungen auch für Immunpräzipitationsverfahren nutzbar zu machen, beziehungsweise ein Reagenz enthaltend eine Mischung von mindestens zwei unterschiedlichen Antikörpern aus der Gruppe monoklonale, gentechnisch hergestellte und synthetische Antikörper und mit Spezifitäten für unterschiedliche Epitope eines Analyten so zu verbessern, dass damit auch in Immunpräzipitationsverfahren eine sensitive und spezifische Bestimmung des Analyten erzielt wird.

Die Aufgabe wird dadurch gelöst, dass bei der Verwendung eines Reagenzes enthaltend eine Mischung von mindestens zwei unterschiedlichen Antikörpern aus der Gruppe monoklonale, gentechnisch hergestellte und synthetische Antikörper und mit Spezifitäten für unterschiedliche Epitope eines Analyten in einem Immunpräzipitationsverfahren dem Reaktionsgemisch zusätzlich ein oder mehrere Immunglobuline ohne Spezifität für den Analyten zugegeben werden.

Dies bewirkt, dass mit nicht-Blut-basierten, multiklonalen Antikörpermischungen in Immunpräzipitationsverfahren eine sensitive und spezifische Bestimmung des Analyten erzielt wird. Auch wenn damit in Immunpräzipitationsverfahren nicht in allen Fällen vollständig auf tierisches Serum bzw. auf aus Tieren gewonnene Komponenten verzichtet werden kann, so ermöglicht die erfindungsgemäße Lösung jedoch den Verzicht auf die Immunisierung von Tieren und auf die Haltung und die Schlachtung immunisierter Tiere, so dass der Tierverbrauch für die Herstellung von Reagenzien für das erfindungsgemäße Verfahren insgesamt auf ein Mindestmaß reduziert wird.

Ein erster Gegenstand der Erfindung ist also ein Immunpräzipitationsverfahren zur Bestimmung eines Analyten in einer Probe, wobei die Probe mit einer Mischung von mindestens zwei unterschiedlichen Antikörpern aus der Gruppe monoklonale, gentechnisch hergestellte und synthetische Antikörper und mit Spezifitäten für unterschiedliche Epitope des Analyten zu einem Reaktionsgemisch vermischt wird, wobei dem Reaktionsgemisch ferner ein oder mehrere Immunglobuline ohne Spezifität für den Analyten zugegeben werden.

Unter einem "Immunpräzipitationsverfahren zur Bestimmung eines Analyten" sind alle Immunoassayformate zu verstehen, die auf dem qualitativen, semi-quantitativen oder quantitativen Nachweis von hochmolekularen Immunpräzipitaten basieren, wobei die Entstehung der Immunpräzipitate auf einer Antigen-Antikörper-Wechselwirkung beruht.

In einer bevorzugten Ausführungsform des erfindungsgemäßen Immunpräzipitationsverfahrens werden zur Bestimmung des Analyten die Immunpräzipitate in einer flüssigen Phase gemessen, indem die Trübung der flüssigen Phase gemessen wird. Besonders bevorzugt wird die Trübung durch die Immunpräzipitate turbidimetrisch gemessen, also durch die Messung des Anstiegs der Extinktion eines flüssigen Reakionsgemischs, oder nephelometrisch, also durch die Messung der Zunahme einer Streulichtintensität unter einem festgelegten Winkel gegenüber einfallendem Licht. Eine bevorzugte Ausführungsform ist also ein nephelometrisches oder turbidimetrisches Immunpräzipitationsverfahren.

Der Begriff "Analyt" umfasst in erster Linie Substanzen, die immunogene Epitope aufweisen, wie z.B. Proteine, Peptide, Nukleinsäuren, Lipide, Lipopolysaccharide etc., und die direkt von einem spezifischen Antikörper gebunden werden können. In diesem Fall handelt es sich bei dem Analyten um ein Antigen, das von einem für dieses Antigen spezifischen Antikörper gebunden werden kann. Typische Beispiele für Analyten, die in der in vitro-Diagnostik von Interesse sind, sind beispielsweise Plasma- oder Serumproteine, wie z.B. Antikörper, die bei bestimmten Krankheitszuständen in abnormal hohen oder niedrigen Konzentrationen vorliegen, oder Bestandteile von Viren oder Bakterien, die das Vorliegen einer Infektion mit einem Krankheitserreger anzeigen.

Der Begriff "Probe" umfasst Körperflüssigkeitsproben insbesondere von Menschen und Tieren, wie beispielsweise Blut, Plasma, Serum, Urin, Speichel oder Nervenwasser, aber auch Stuhlproben oder Proben von Geweben. Gegebenenfalls müssen die Proben vorbehandelt werden, um den Analyten für das Nachweisverfahren zugänglich zu machen oder um störende Probenbestandteile zu entfernen. Solch eine Vorbehandlung von Proben kann z.B. die Abtrennung und/oder Lyse von Zellen beinhalten oder die Zentrifugation von Proben.

Erfindungsgemäß wird die Probe mit einer Mischung von zwei oder mehr unterschiedlichen Antikörpern mit Spezifitäten für unterschiedliche Epitope des Analyten zu einem Reaktionsgemisch vermischt.

Bevorzugterweise wird die Probe mit einer Mischung von mehr als zwei unterschiedlichen Antikörpern mit Spezifitäten für unterschiedliche Epitope des Analyten zu einem Reaktionsgemisch vermischt, besonders bevorzugt mit drei, vier, fünf, sechs oder mehr solcher unterschiedlicher Antikörper. Zur Nachahmung von polyklonalen Seren haben sich Mischungen von vier bis sechs unterschiedlichen Antikörpern bewährt.

Die Analyt-spezifischen Antikörper sind ausgewählt aus der Gruppe monoklonale, gentechnisch hergestellte und synthetische Antikörper; diese können weitestgehend ohne Tierverbrauch gewonnen werden.

Unter einem "monoklonalen Antikörper" ist ein Antikörper zu verstehen, der von einer Zelllinie produziert wird, die auf eine einzelne, isolierte Zelle zurückgeht. Derartige Zelllinien werden typischerweise mit Hilfe von Hybridom-Techniken etabliert, bei denen Immunzellen immunisierter Tiere, wie z.B. einer Maus oder eines Kaninchens, mit Myelomzellen zum Erzeugen von Antikörper-produzierenden Hybridomazellen verschmolzen werden und anschließend geeignete Klone isoliert und vereinzelt werden. Die so gewonnenen Hybridomazelllinien stehen dann für eine dauerhafte Produktion von monoklonalen Antikörpern zur Verfügung. Größere Antikörpermengen lassen sich beispielsweise aus dem Zellkulturüberstand aus Fermentern oder Rollerkulturen gewinnen.

Unter einem "gentechnisch hergestellten Antikörper" (auch "rekombinanter Antikörper" genannt) ist ein Antikörper zu verstehen, der von einer gentechnisch veränderten Zelllinie produziert wird. Derartige Zelllinien werden typischerweise etabliert durch die Transformation oder Transfektion von isolierten tierischen, menschlichen, pflanzlichen, pilzlichen oder prokaryontischen Zellen mit einer Nukleinsäure, die den Antikörper codiert. Für das Auffinden von Nukleinsäuresequenzen, die Antigen-spezifische Antikörper oder Fragmente davon codieren, eignen sich die bekannten Phage-Display Techniken. Die codierenden Nukleinsäuresequenzen werden dann in geeignete Vektoren/Plasmide kloniert, die dann in die Wirtszelle eingeschleust werden. Die so gewonnenen gentechnisch veränderten Zelllinien stehen dann für eine dauerhafte Produktion von gentechnisch hergestellten Antikörpern zur Verfügung. Größere Antikörpermengen lassen sich beispielsweise aus dem Zellkulturüberstand aus Fermentern oder Rollerkulturen gewinnen.

Unter einem "synthetischen Antikörper" ist ein Antikörper zu verstehen, der mit einer zellfreien Methode produziert wird. In Kenntnis der Aminosäuresequenz eines geeigneten Antikörpers kann dieser in vitro per chemischer Merrifield-Synthese von Peptiden und anschließender Proteinligation der Peptide erzeugt werden. Eine andere Methode ist die zellfreie Genexpression, bei der auf Grundlage der bekannten Aminosäuresequenz/DNA-Sequenz eines geeigneten Antikörpers, z.B. mittels in vitro-Transkription, eine Antikörpercodierende RNA erzeugt wird, die dann mit einer Aminosäuremischung in einem Lysat oder Extrakt (sog. "Translationsextrakte") aus z.B. E. coli, Insektenzellen, Weizenkeimen oder CHO-Zellen inkubiert wird. Ein so erzeugter Antikörper lässt sich dann aus dem Gemisch gewinnen.

Ein Analyt-spezifischer Antikörper aus der Gruppe monoklonale, gentechnisch hergestellte und synthetische Antikörper ist typischerweise ein Immunglobulin, zum Beispiel ein Immunglobulin (Ig) der Klasse bzw. Subklasse IgA, IgD, IgE, IgG₁, IgG₂ₐ, IgG_{2b}, IgG₃, IgG₄ oder IgM. Er weist mindestens eine Bindungsstelle (häufig Paratop genannt) für ein Epitop (häufig auch antigene Determinante genannt) auf einem Antigen auf. Ein solches Epitop ist zum Beispiel durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion funktioniert nach dem sogenannten "Schlüssel-Schloss-Prinzip" und ist in der Regel in einem hohen Grad spezifisch, d.h. die Antikörper vermögen kleine Abweichungen in der Primärstruktur, in der Ladung, in der räumlichen Konfiguration und der sterischen Anordnung des Antigens zu unterscheiden. Insbesondere die sogenannten "complementarity determining regions" des Antikörpers tragen zur Bindung des Antikörpers an das Antigen bei. Der Antikörper kann vollständig einem Antikörper entsprechen, der ursprünglich z.B. durch Immunisierung eines Menschen oder eines Tieres, wie z.B. Maus, Ratte, Meerschweinchen, Kaninchen, Pferd, Esel, Kamel, Schaf, Ziege oder Huhn, als Teil der natürlichen Immunantwort produziert wurde und nun durch Hybridomzelltechniken oder Klonierung und Expression der codierenden Nukleinsäuresequenz reproduziert wird. Alternativ können auch mittels gentechnischer Methoden modifizierte Antikörper hergestellt werden, die durch eine Kombination Immunglobulin-codierender Sequenzen verschiedener Spezies gebildet werden ("chimäre Antikörper"); z.B. die variable Region eines Maus-Antikörpers wird mit der konstanten Region eines humanen oder eines Kaninchen Antikörpers verknüpft.

Erfindungsgemäß wird dem Reaktionsgemisch ferner ein Immunglobulin oder mehrere Immunglobuline ohne Spezifität für den Analyten zugegeben.

Es wurde gefunden, dass die Zugabe mindestens eines Immunglobulins ohne Spezifität für den Analyten beziehungsweise die Zugabe mehrerer Immmunglobuline ohne Spezifität für den Analyten, die Entstehung hochmolekularer Immunpräzipitate in dem Reaktionsgemisch begünstigt und damit die Sensitivität des Verfahrens insbesondere in Proben mit geringer Analytkonzentration verbessert.

Der Begriff "Immunglobuline ohne Spezifität für den Analyten" bezeichnet Immunglobuline (oder synonym "Antikörper"), die - im Gegensatz zu den weiter oben beschriebenen Analyt-spezifischen Antikörpern- keine Spezifität für den Analyten aufweisen, den Analyten also nicht spezifisch binden. Sie sind typischerweise erhältlich aus menschlichen beziehungsweise tierischen Seren von Individuen, die nicht mit dem Analyten immunisiert wurden und daher keine Analyt-spezifischen Antikörper produzieren, beziehungsweise aus Zelllinien oder zellfreien Expressionssystemen, die menschliche, tierische oder chimäre Antikörper produzieren, die den Analyten bekanntermaßen nicht spezifisch binden.

In einer Ausführungsform des erfindungsgemäßen Immunpräzipitationsverfahrens ist das eine Immunglobulin beziehungsweise sind die mehreren Immunglobuline ohne Spezifität für den Analyten, das/die dem Reaktionsgemisch zugegeben wird/werden, ausgewählt aus der Gruppe der Immunglobulinklassen bzw. -subklassen IgA, IgD, IgE, IgG₁, IgG₂ₐ, IgG_{2b}, IgG₃, IgG₄ und IgM. Im Falle der Zugabe einer Mischung von Immunglobulinen können diese in jeder denkbaren Kombination hinsichtlich der Immunglobulinklassen in der Mischung enthalten sein.

Ein Immunglobulin ohne Spezifität für den Analyten kann ein aus humanem oder tierischem Serum isoliertes Immunglobulin sein oder ein monoklonales Immunglobulin (analog einem monoklonalen Antikörper wie weiter oben beschrieben) oder ein gentechnisch hergestelltes Immunglobulin (analog einem gentechnisch hergestellten Antikörper wie weiter oben beschrieben) oder ein synthetisches Immunglobulin (analog einem synthetischen Antikörper wie weiter oben beschrieben).

In einer weiteren Ausführungsform des erfindungsgemäßen Immunpräzipitationsverfahrens wird dem Reaktionsgemisch genau ein Immunglobulin ohne Spezifität für den Analyten zugegeben, beispielsweise ein humanes oder tierisches Immunglobulin G (IgG), M (IgM), A (IgA), E (IgE) oder D (IgD), besonders bevorzugt aus Kaninchen oder Maus. Besonders bevorzugterweise wird in diesem Fall ein gentechnisch hergestelltes Immunglobulin verwendet, was den Vorteil hat, dass das gesamte Immunpräzipitationsverfahrens ohne Verwendung Blut-basierter Antikörper durchführbar ist.

In einer weiteren Ausführungsform des erfindungsgemäßen Immunpräzipitationsverfahrens wird dem Reaktionsgemisch eine Mischung mehrerer Immunglobuline ohne Spezifität für den Analyten zugegeben, beispielsweise eine Mischung von Immunglobulinen ausgewählt aus der Gruppe IgA, IgD, IgE, IgG₁, IgG₂ₐ, IgG_{2b}, IgG₃, IgG₄ und IgM, wiederum ausgewählt aus humanen und tierischen Immunglobulinen, besonders bevorzugt aus Kaninchen oder Maus. Besonders bevorzugterweise werden in diesem Fall teilweise, ganz besonders bevorzugt ausschließlich gentechnisch hergestellte Immunglobuline verwendet, was den Vorteil hat, dass das gesamte Immunpräzipitationsverfahrens in reduziertem Umfang beziehungsweise ganz ohne Verwendung Blut-basierter Antikörper durchführbar ist.

In einer anderen Ausführungsform des erfindungsgemäßen Immunpräzipitationsverfahrens besteht die Mischung aus mehreren Immunglobulinen ohne Spezifität für den Analyten, die dem Reaktionsgemisch zugegeben wird, aus einem humanen oder tierischen Normalserum oder aus einer Mischung von humanem und einem oder mehreren tierischen Normalseren oder aus einer Mischung verschiedener tierischer Normalseren. Bevorzugte tierische Normalseren stammen aus Kaninchen oder Maus. Im Falle der Zugabe einer Mischung von Normalseren können diese in jeder denkbaren Kombination hinsichtlich der Herkunftsspezies (insbesondere Mensch, Maus, Kaninchen) und zu unterschiedlichen oder gleichen Anteilen in der Mischung enthalten sein. Unter einem Normalserum sind auch stabilisierte Seren zu verstehen, die zu diesem Zweck filtriert oder anderweitig vorbehandelt wurden.

In noch einer anderen Ausführungsform des erfindungsgemäßen Immunpräzipitationsverfahrens besteht die Mischung aus mehreren Immunglobulinen ohne Spezifität für den Analyten, die dem Reaktionsgemisch zugegeben wird, aus einer gereinigten Präparation von Immunglobulinen einer oder mehrerer Immunglobulinklassen aus humanem Serum oder aus tierischem Serum oder aus einer Mischung solcher gereinigter Präparationen. Bevorzugte gereinigte Präparationen von Immunglobulinen sind beispielsweise Gesamt-IgG-Präparationen, bevorzugt aus Kaninchen- oder Mausserum. Verschiedene Verfahren zur Gewinnung von Immunglobulinen aus Plasma oder Serum sind hinreichend bekannt. Beispielsweise kann Immunglobulin G mittels eines Alkoholfällungsverfahrens angereichert werden (Cohn, E.J. et al., Preparation and Properties of Serum and Plasma Proteins. IV. A System for the Separation into Fractions of the Protein and Lipoprotein Components of Biological Tissues and Fluids. J. Am. Chem. Soc. 68, 459-475, 1946; und Cohn, E.J. et al., A System for the Separation of the Components of Human Blood: Quantitative Procedures for the Separation of the Protein Components of Human Plasma. J. Am. Chem. Soc. 72, 465-474, 1950; und US 3,597,409). Eine weitere bekannte Methode besteht in der Kombination von Fällungsschritten mit Rivanol und Ammoniumsulfat (Horejisi, J. and Smetana, R., The Isolation of Gamma Globulin from Blood-Serum by Rivanol. Acta Med. Scand. Vol. 155, 65-70, 1956).

In einer bevorzugten Ausführungsform des erfindungsgemäßen Immunpräzipitationsverfahrens werden die Mischung von mindestens zwei unterschiedlichen Antikörpern mit Spezifitäten für unterschiedliche Epitope des Analyten und das eine oder die mehreren Immunglobuline ohne Spezifität für den Analyten gleichzeitig, in Form eines einzigen Reagenzes mit der Probe vermischt. Ein solches Reagenz ist weiter unten genauer beschrieben.

Nichtsdestotrotz ist das erfindungsgemäße Immunpräzipitationsverfahrens grundsätzlich auch so durchführbar, dass in einem ersten Schritt a) die Probe mit der Mischung von mindestens zwei unterschiedlichen Antikörpern aus der Gruppe monoklonale, gentechnisch hergestellte und synthetische Antikörper und mit Spezifitäten für unterschiedliche Epitope des Analyten zu einem Reaktionsgemisch vermischt wird, und dann in einem zweiten Schritt b) dem Reaktionsgemisch ein oder mehrere Immunglobuline ohne Spezifität für den Analyten zugegeben werden. Es ist ferner möglich, die beiden Schritte a) und b) in umgekehrter zeitlicher Reihenfolge durchzuführen.

Ein weiterer Gegenstand der vorliegenden Erfindung ist die Verwendung eines oder mehrerer Immunglobuline ohne Spezifität für den Analyten zur Verstärkung der
Immunpräzipitationsreaktion in einem
Immunpräzipitationsverfahrens zur Bestimmung des Analyten in einer Probe.

Noch ein weiterer Gegenstand der Erfindung ist ein Reagenz zur Verwendung in einem Immunpräzipitationsverfahren zur Bestimmung eines Analyten, wobei das Reagenz eine Mischung von mindestens zwei unterschiedlichen Antikörpern aus der Gruppe monoklonale, gentechnisch hergestellte und synthetische Antikörper und mit Spezifitäten für unterschiedliche Epitope des Analyten und zusätzlich ein oder mehrere Immunglobuline ohne Spezifität für den Analyten enthält.

Bei dem Reagenz kann es sich um eine flüssige Lösung oder ein resuspendierbares Lyophilisats einer solchen Lösung handeln.

Die in dem Reagenz enthaltenen Analyt-spezifischen Antikörper sind ausgewählt aus der Gruppe monoklonale, gentechnisch hergestellte und synthetische Antikörper, wie bereits weiter oben erläutert.

Ein bevorzugtes Reagenz enthält eine Mischung von zwei, drei, vier, fünf, sechs oder mehr unterschiedlichen Antikörpern mit Spezifitäten für unterschiedliche Epitope des Analyten.

Das eine oder die mehreren in dem Reagenz enthaltenen Immunglobuline ohne Spezifität für den Analyten (wie weiter oben näher erläutert) sind bevorzugterweise ausgewählt aus der Gruppe der Immunglobulinklassen bzw. -subklassen IgA, IgD, IgE, IgG₁, IgG₂ₐ, IgG_{2b}, IgG₃, IgG₄ und IgM.

Das eine oder die mehreren in dem Reagenz enthaltenen Immunglobuline ohne Spezifität für den Analyten können aus humanem oder tierischem Serum isolierte, monoklonale, gentechnisch hergestellte oder synthetische Immunglobuline sein.

Das eine oder die mehreren in dem Reagenz enthaltenen Immunglobuline ohne Spezifität für den Analyten können aus Mensch, Maus oder Kaninchen stammen.

In einer Ausführungsform des Reagenzes enthält dieses genau ein Immunglobulin ohne Spezifität für den Analyten.

In einer anderen Ausführungsform des Reagenzes enthält dieses eine Mischung mehrerer Immunglobuline ohne Spezifität für den Analyten. Die Mischung mehrerer Immunglobuline ohne Spezifität für den Analyten kann aus einem humanen oder tierischen Normalserum oder einer Mischung davon bestehen (wie auch weiter oben beschrieben), oder sie kann aus einer gereinigten Präparation von Immunglobulinen einer oder mehrerer Immunglobulinklassen aus humanem Serum oder aus tierischem Serum oder aus einer Mischung solcher gereinigter Präparationen bestehen.

Ein besonders bevorzugtes Reagenz enthält zusätzlich ein polares Lösungsmittel, vorzugsweise γ-Butyrolactam (2-Pyrrolidon), weil es dann im flüssigen Zustand besonders stabil ist. Bevorzugterweise enthält ein flüssiges Reagenz bis zu 10 Volumenprozent des polaren Lösungsmittels.

Ein weiterer Gegenstand der vorliegenden Erfindung ist die Verwendung eines erfindungsgemäßen Reagenzes zur Durchführung eines Immunpräzipitationsverfahrens zur Bestimmung eines Analyten in einer Probe. Die Verwendung eines erfindungsgemäßen Reagenzes ermöglicht eine vereinfachte Durchführung eines Immunpräzipitationsverfahrens zur Bestimmung eines Analyten in einer Probe, indem es die simultane Zugabe der Mischung von mindestens zwei unterschiedlichen Antikörpern mit Spezifitäten für unterschiedliche Epitope des Analyten und des einen oder der mehreren Immunglobuline ohne Spezifität für den Analyten ermöglicht.

Die folgenden Beispiele und die Figuren dienen der Veranschaulichung der vorliegenden Erfindung und sind nicht als Einschränkung zu verstehen.

Unabhängig vom grammatikalischen Geschlecht eines bestimmten Begriffes sind Personen mit männlicher, weiblicher oder anderer Geschlechtsidentität mit umfasst.

### BEISPIELE

| | |
|---|---|
| **BEISPIEL 1:** | **Herstellung eines erfindungsgemäßen anti-Human-IgG-Reagenzes enthaltend eine Mischung aus verschiedenen monoklonalen anti-Human-IgG-Antikörpern und tierischem Serum bzw. gereinigten tierischen Immunglobulinen und Verwendung in einem Immunpräzipitationsverfahren zur Bestimmung von Human-IgG in Serumproben** |
| **Beispiel 1a):** | **Herstellung der Reagenzien** |

Jeweils 1 mL einer Mischung von vier unterschiedlichen monoklonalen Antikörpern (produziert von vier unterschiedlichen Maus-Hybridomazelllinien) ("MAK-Mix"), die vier unterschiedliche Epitope auf dem Fc-Teil der schweren Kette von humanem Immunglobulin G (IgG) spezifisch binden, in PBS-Puffer, pH 7,4 (Gesamtproteinkonzentration 2 mg/mL) wurde mit jeweils 1 mL
i) eines über einen 0,22 um Millex-GV PVDF Filter (Merck Millipore Ltd.) vorfiltrierten Rohnormalserums aus Kaninchen; oder
ii) eines über einen 0,22 um Millex-GV PVDF Filter (Merck Millipore Ltd.) vorfiltrierten Rohnormalserums aus Maus;
iii) PBS-Puffer, pH 7,4 (also kein Zusatz von unspezifischen Immunglobulinen); oder
iv) einer BSA-Lösung (40 mg/mL bovines Serumalbumin in PBS-Puffer, pH 7,4) (also kein Zusatz von unspezifischen Immunglobulinen); oder
v) eines über einen 0,22 um Millex-GV PVDF Filter (Merck Millipore Ltd.) vorfiltrierten Rohnormalserums vom Rind;
vi) einer Gesamt-Immunglobulin-Präparation aus Kaninchenserum;
vii) einer Immunglobulin G-Präparation aus Kaninchenserum; oder
viii) einer Mischung rekombinanter Immunglobuline G mit Kaninchen Fc-Backbone

vermischt und durch Zugabe von 2-Pyrrolidon (Endkonzentration 1,5 Volumenprozent) stabilisiert.

Die Immunglobulin G-Präparation aus Kaninchenserum wurde mittels Ionenaustausch-Chromatographie mit DEAE Sepharose aus einer Gesamt-Immunglobulin-Präparation gewonnen, wie von Fishman, J.B. und Berg, E.A. 2019 beschrieben (Purification of Antibodies: Diethylaminoethyl (DEAE)Chromatography, Cold Spring Harb Protoc; doi:10.1101/pdb.prot099135).

| | |
|---|---|
| **Beispiel 1b):** | **Nephelometrischer Immunpräzipitationsassay zur Bestimmung von Human-IgG in Serumproben** |

Als Probenmaterial wurden Serumproben von sechs gesunden humanen Spendern (Normalseren, NS 1-6) mit Human-IgG-Konzentrationen im Referenzbereich (7-16 g/L) und zwei Mangelserumproben (MS 1 und 2) (jeweils 0 g/l Human-IgG) ohne Zusatz und mit Zusatz unterschiedlicher Mengen Human-IgG durch Zugabe von Privigen Infusionslösung (100 mg/mL normales Immunglobulin vom Menschen, CSL Behring, Marburg) verwendet.

Die Durchführung der Assays erfolgte automatisch als Endpunkttest auf dem BN II System (Siemens Healthineers). Dazu werden die Proben vom System zunächst 1:400 mit einer Verdünnungslösung (N-Diluens, Siemens Healthineers) verdünnt. 100 µL der so verdünnten Probe werden dann zunächst mit 160 µL eines Reaktionspuffers (N-Reaktionspuffer, Siemens Healthineers) und anschließend mit 40 µL eines anti-Human-IgG-Reagenzes (siehe Reagenzien i) bis v) in Beispiel 1a)) in einer Reaktionsküvette vermischt. Die resultierende Lichtstreuung wird über einen Zeitraum von 360 Sekunden bei +37 °C gemessen. Die so gemessenen Signalrohwerte (Signal [Bit]) stellen die Messergebnisse dar.

Die Ergebnisse wurden mit den Ergebnissen des kommerziell erhältlichen N-Antiserum Assays (Siemens Healthineers, Siemens Materialnummer (SMN) 10446296) zur Bestimmung von Human-IgG, der ebenfalls auf dem BN II System automatisch durchgeführt wurde, verglichen. Der N-Antiserum Assay zur Bestimmung von Human-IgG umfasst als Nachweisreagenz ein flüssiges polyklonales Kaninchenserum (gewonnen aus mit hochgereinigtem Human-IgG immunisierten Kaninchen).

Die Ergebnisse sind in Tabelle 1 und Tabelle 2 dargestellt.

In Tabelle 1 wird deutlich, dass der MAK-Mix mit Zusatz von Maus- oder Kaninchen-Normalserum eine vierfach höhere Sensitivität für die Detektion von Human-IgG zeigt als der MAK-Mix ohne Zusatz oder mit Zusatz von Rinder-Normalserum. Der MAK-Mix mit Zusatz von Maus- oder Kaninchen-Normalserum zeigt eine Sensitivität, die vergleichbar hoch ist wie die Sensitivität des kommerziell erhältlichen N Antiserum Assays. Ohne den erfindungsgemäßen Zusatz von Maus- oder Kaninchen-Normalserum wird zumindest im unteren Human-IgG-Konzentrationsbereich keine ausreichende Sensitivität mit dem MAK-Mix erzielt.

**Tabelle 1: Vergleich der Testergebnisse bei der Verwendung unterschiedlicher anti-Human-IgG-MAK-Mix-Reagenzien enthaltend tierische Seren**

| | | **MAK-MIX** | | | | |
|---|---|---|---|---|---|---|
| **Probe - Human-IgG [mg/mL]** | **N Antiserum gegen Human-IgG** | **ohne Zusatz** | **mit BSA** | **mit Rinder-Normalserum** | **mit Kaninchen -Normalserum** | **mit Maus-Normal -serum** |
| **Signal MW [Bit]** | | | | | | |
| NS 1 | 2421 | 989 | 966 | 979 | 2809 | 2608 |
| NS 2 | 949 | 47 | 45 | 40 | 921 | 839 |
| NS 3 | 2611 | 1281 | 1322 | 1304 | 3182 | 2955 |
| NS 4 | 3111 | 2146 | 2099 | 2216 | 4313 | 3981 |
| NS 5 | 2327 | 962 | 951 | 948 | 2693 | 2501 |
| NS 6 | 2538 | 1241 | 1191 | 1199 | 3059 | 2827 |
| | | | | | | |
| MS 1 | 1 | 4 | 6 | 9 | 7 | 5 |
| MS 1 - 0,002 | 29 | 13 | 5 | 11 | 23 | 19 |
| MS 1 - 0,004 | 138 | 24 | 12 | 5 | 125 | 114 |
| MS 1 - 0,007 | 318 | 5 | 3 | 10 | 299 | 265 |
| MS 1 - 0,014 | 889 | 44 | 39 | 31 | 847 | 786 |
| MS 1 - 0,029 | 1678 | 267 | 265 | 233 | 1601 | 1481 |
| MS 1 - 0,058 | 2875 | 2120 | 2281 | 2184 | 3452 | 3209 |
| MS 1 - 0,116 | 4012 | 5760 | 6038 | 5819 | 7241 | 6724 |
| | | | | | | |
| MS 2 | 4 | 2 | 7 | -2 | 6 | 5 |
| MS 2 - 0,002 | 23 | 2 | 2 | 9 | 29 | 26 |
| MS 2 - 0,004 | 129 | 19 | 5 | 11 | 131 | 109 |
| MS 2 - 0,007 | 324 | 11 | 7 | 5 | 305 | 299 |
| MS 2 - 0,014 | 899 | 27 | 25 | 24 | 826 | 757 |
| MS 2 - 0,029 | 1601 | 249 | 251 | 253 | 1653 | 1531 |
| MS 2 - 0,058 | 2801 | 2201 | 2093 | 2007 | 3423 | 3170 |
| MS 2 - 0,116 | 3897 | 5978 | 5715 | 5991 | 7071 | 6289 |

In Tabelle 2 wird deutlich, dass der MAK-Mix mit Zusatz von gereinigten Kaninchen-Immunglobulinen (Gesamt-Immunglobulin- und Immunglobulin G-Präparationen) oder einer Mischung von rekombinanten Immunglobulinen G mit Kaninchen Fc-Backbone eine siebenfach höhere Sensitivität für die Detektion von Human-IgG zeigt als der MAK-Mix ohne Zusatz. Der MAK-Mix mit Zusatz der verschiedenen Kaninchen-Immunglobuline zeigt eine Sensitivität, die vergleichbar hoch ist wie die Sensitivität des kommerziell erhältlichen N Antiserum Assays. Ohne den erfindungsgemäßen Zusatz von Kaninchen-Immunglobulinen wird zumindest im unteren Human-IgG-Konzentrationsbereich keine ausreichende Sensitivität mit dem MAK-Mix erzielt.

**Tabelle 2: Vergleich der Testergebnisse bei der Verwendung unterschiedlicher anti-Human-IgG-MAK-Mix-Reagenzien enthaltend Kaninchen-Immunglobuline**

| | | **MAK-MIX** | | | |
|---|---|---|---|---|---|
| **Probe - Human-IgG [mg/mL]** | **N Antiserum gegen Human-IgG** | **ohne Zusatz** | **mit Gesamt-Immunglobulin aus Kaninchen** | **mit Immunglobulin G aus Kaninchen** | **mit rek. Immunglobulinen G mit Kaninchen Fc-Backbone** |
| **Signal MW [Bit]** | | | | | |
| NS 1 | 2421 | 989 | 2481 | 2466 | 2576 |
| NS 2 | 949 | 49 | 901 | 933 | 966 |
| NS 3 | 2611 | 1281 | 2666 | 2747 | 2723 |
| NS 4 | 3111 | 2146 | 3626 | 3701 | 3769 |
| NS 5 | 2327 | 962 | 2455 | 2528 | 2554 |
| NS 6 | 2538 | 1241 | 2810 | 2921 | 3001 |
| | | | | | |
| MS 1 | 1 | 4 | 5 | 7 | 4 |
| MS 1 - 0,002 | 29 | 13 | 50 | 54 | 61 |
| MS 1 - 0,004 | 138 | 4 | 181 | 190 | 202 |
| MS 1 - 0,007 | 318 | 5 | 346 | 389 | 368 |
| MS 1 - 0,014 | 889 | 44 | 871 | 903 | 922 |
| MS 1 - 0,029 | 1678 | 267 | 1655 | 1834 | 1902 |
| MS 1 - 0,058 | 2875 | 2120 | 3507 | 3522 | 3589 |
| MS 1 - 0,116 | 4012 | 5760 | 7402 | 7389 | 7289 |
| | | | | | |
| MS 2 | 4 | 2 | 4 | 6 | 6 |
| MS 2 - 0,002 | 23 | 2 | 53 | 50 | 54 |
| MS 2 - 0,004 | 129 | 1 | 192 | 185 | 188 |
| MS 2 - 0,007 | 324 | 11 | 375 | 378 | 381 |
| MS 2 - 0,014 | 899 | 27 | 853 | 863 | 891 |
| MS 2 - 0,029 | 1601 | 249 | 1709 | 1787 | 1866 |
| MS 2 - 0,058 | 2801 | 2201 | 3522 | 3493 | 3541 |
| MS 2 - 0,116 | 3897 | 5978 | 7266 | 7215 | 7173 |

| | |
|---|---|
| **BEISPIEL 2:** | **Herstellung eines erfindungsgemäßen anti-Human-IgG-Reagenzes enthaltend eine Mischung aus verschiedenen rekombinanten anti-Human-IgG-Antikörpern und gereinigten Maus- und Human-Immunglobulinen und Verwendung in einem Immunpräzipitationsverfahren zur Bestimmung von Human-IgG in Serumproben** |
| **Beispiel 2a):** | **Herstellung der Reagenzien** |

Jeweils 1 mL einer Mischung von vier rekombinanten, gentechnisch hergestellten Antikörpern ("Rek. MAK-Mix"), die vier unterschiedliche Epitope auf dem Fc-Teil der schweren Kette von humanem Immunglobulin G (IgG) spezifisch binden, in PBS-Puffer, pH 7,4 (Gesamtproteinkonzentration 5 mg/mL) wurde mit jeweils 1 mL
i) einer Immunglobulin G-Präparation aus Mausserum (0,5 mg/mL); oder
ii) einer Immunglobulin-Präparation aus humanem Serum (Privigen Infusionslösung, 0,05 mg/mL normales Immunglobulin vom Menschen, CSL Behring, Marburg)
vermischt.

Die Antigen-bindenden Domänen der vier rekombinanten antihuman-IgG-Fc-Antikörper waren mittels der Phagen-Display-Technologie aus einer rekombinanten Phagen-Bibliothek enthaltend die variablen Teile von Immunglobulin Leicht- und Schwerketten isoliert worden und wurden anschließend in ein Kaninchen-IgG-Backbone kloniert. Jedes Konstrukt wurde anschließend in einem HEK-Zellexpressionssystem exprimiert.

| | |
|---|---|
| **Beispiel 2b):** | **Nephelometrischer Immunpräzipitationsassay zur Bestimmung von Human-IgG in Serumproben** |

Die nephelometrische Bestimmung von Human-IgG wurde wie in Beispiel 1b) beschrieben durchgeführt.

Die Ergebnisse sind in Tabelle 3 dargestellt.

In Tabelle 3 wird deutlich, dass der Rek. MAK-Mix mit Zusatz von Maus-IgG oder selbst mit einer niedrig dosierten Menge Human-Ig eine doppelt so hohe Sensitivität für die Detektion von Human-IgG zeigt wie der Rek. MAK-Mix ohne Zusatz.

**Tabelle 3: Vergleich der Testergebnisse bei der Verwendung unterschiedlicher anti-Human-IgG-Rek. MAK-Mix-Reagenzien enthaltend Immunglobuline aus Maus oder Mensch**

| | **Rek. MAK-MIX** | | |
|---|---|---|---|
| **Probe - Human-IgG [mg/mL]** | **ohne Zusatz** | **mit Maus-IgG** | **mit Human-Ig** |
| **Signal MW [Bit]** | | | |
| NS 1 | 989 | 2481 | 2466 |
| NS 2 | 49 | 901 | 933 |
| NS 3 | 1281 | 2666 | 2747 |
| NS 4 | 2146 | 3626 | 3701 |
| NS 5 | 962 | 2455 | 2528 |
| NS 6 | 1241 | 2810 | 2921 |
| | | | |
| MS 1 | 4 | 5 | 7 |
| MS 1 - 0,002 | 13 | 50 | 54 |
| MS 1 - 0,004 | 4 | 181 | 190 |
| MS 1 - 0,007 | 5 | 346 | 389 |
| MS 1 - 0,014 | 44 | 871 | 903 |
| MS 1 - 0,029 | 267 | 1655 | 1834 |
| MS 1 - 0,058 | 2120 | 3507 | 3522 |
| MS 1 - 0,116 | 5760 | 7402 | 7389 |
| | | | |
| MS 2 | 2 | 4 | 6 |
| MS 2 - 0,002 | 2 | 53 | 50 |
| MS 2 - 0,004 | 1 | 192 | 185 |
| MS 2 - 0,007 | 11 | 375 | 378 |
| MS 2 - 0,014 | 27 | 853 | 863 |
| MS 2 - 0,029 | 249 | 1709 | 1787 |
| MS 2 - 0,058 | 2201 | 3522 | 3493 |
| MS 2 - 0,116 | 5978 | 7266 | 7215 |

## Patentansprüche

1. Reagenz zur Verwendung in einem
Immunpräzipitationsverfahren zur Bestimmung eines Analyten, das Reagenz enthaltend eine Mischung von mindestens zwei unterschiedlichen Antikörpern aus der Gruppe monoklonale, gentechnisch hergestellte und synthetische Antikörper und mit Spezifitäten für unterschiedliche Epitope des Analyten, **dadurch gekennzeichnet, dass** das Reagenz zusätzlich ein oder mehrere Immunglobuline ohne Spezifität für den Analyten enthält.

2. Reagenz gemäß Anspruch 1, enthaltend eine Mischung von drei, vier, fünf, sechs oder mehr unterschiedlichen Antikörpern mit Spezifitäten für unterschiedliche Epitope des Analyten.

3. Reagenz gemäß einem der vorhergehenden Ansprüche, wobei das eine oder die mehreren Immunglobuline ohne Spezifität für den Analyten ausgewählt sind aus der Gruppe der Immunglobulinklassen bzw. -subklassen Immunglobulin G (IgG), G1 (IgG₁), G2a (IgG₂ₐ), G2b (IgG_{2b}), G3 (IgG₃), G4 (IgG₄), M (IgM), A (IgA), E (IgE) und D (IgD).

4. Reagenz gemäß einem der vorhergehenden Ansprüche, wobei das eine oder die mehreren Immunglobuline ohne Spezifität für den Analyten aus humanem oder tierischem Serum isolierte, monoklonale, gentechnisch hergestellte oder synthetische Immunglobuline sind.

5. Reagenz gemäß einem der vorhergehenden Ansprüche, das genau ein Immunglobulin ohne Spezifität für den Analyten enthält.

6. Reagenz gemäß einem der Ansprüche 1 bis 4, das eine Mischung mehrerer Immunglobuline ohne Spezifität für den Analyten enthält.

7. Reagenz gemäß Anspruch 6, wobei die Mischung mehrerer Immunglobuline ohne Spezifität für den Analyten aus einem humanen oder tierischen Normalserum oder aus einer Mischung von humanem und tierischem Normalserum oder aus einer Mischung verschiedener tierischer Normalseren besteht.

8. Reagenz gemäß Anspruch 6, wobei die Mischung mehrerer Immunglobuline ohne Spezifität für den Analyten aus einer gereinigten Präparation von Immunglobulinen einer oder mehrerer Immunglobulinklassen aus humanem Serum oder aus tierischem Serum oder aus einer Mischung solcher gereinigter Präparationen besteht.

9. Reagenz gemäß einem der vorhergehenden Ansprüche, wobei das eine oder die mehreren Immunglobuline ohne Spezifität für den Analyten aus Mensch, Maus oder Kaninchen stammen.

10. Reagenz gemäß einem der vorhergehenden Ansprüche, ferner enthaltend ein polares Lösungsmittel, vorzugsweise γ-Butyrolactam.

11. Verwendung eines Reagenzes gemäß einem der Ansprüche 1 bis 10 zur Durchführung eines Immunpräzipitationsverfahrens zur Bestimmung eines Analyten in einer Probe.

12. Immunpräzipitationsverfahren zur Bestimmung eines Analyten in einer Probe, wobei die Probe mit einer Mischung von mindestens zwei unterschiedlichen Antikörpern aus der Gruppe monoklonale, gentechnisch hergestellte und synthetische Antikörper und mit Spezifitäten für unterschiedliche Epitope des Analyten zu einem Reaktionsgemisch vermischt wird, **dadurch gekennzeichnet, dass** dem Reaktionsgemisch ferner ein oder mehrere Immunglobuline ohne Spezifität für den Analyten zugegeben werden.

13. Immunpräzipitationsverfahren gemäß Anspruch 12, wobei die Mischung von mindestens zwei unterschiedlichen Antikörpern mit Spezifitäten für unterschiedliche Epitope des Analyten und das eine oder die mehreren Immunglobuline ohne Spezifität für den Analyten gleichzeitig, in Form eines einzigen Reagenzes mit der Probe vermischt werden.

14. Immunpräzipitationsverfahren gemäß Anspruch 13, wobei ein Reagenz gemäß einem der Ansprüche 1 bis 10 mit der Probe vermischt wird.

15. Immunpräzipitationsverfahren gemäß einem der Ansprüche 12 bis 14, wobei zur Bestimmung des Analyten in der Probe die in dem Reaktionsgemisch entstehenden Immunpräzipitate turbidimetrisch oder nephelometrisch gemessen werden.
